# EUROPEAN PATENT SPECIFICATION

(11) **EP 4 604 954 B1**
(45) Date of publication and mention of the grant of the patent: **16.09.2026**
(21) Application number: 23792922.9
(22) Date of filing: 16.10.2023
(51) Int. Cl.: A61K 31/4425, A61P 31/06, A61P 31/10

(54) **PYRIDINE-2-THIOL 1-OXIDE DERIVATIVES AND THEIR USE FOR TREATMENT OF MAMMALIAN INFECTIONS CAUSED BY MYCOBACTERIUM OR FUNGI**
PYRIDIN-2-THIOL 1-OXID-DERIVATE UND IHRE VERWENDUNG ZUR BEHANDLUNG VON DURCH MYCOBACTERIUM ODER PILZE VERURSACHTEN INFEKTIONEN BEI SÄUGETIEREN
DÉRIVÉS DE PYRIDINE-2-THIOL 1-OXYDE ET LEUR UTILISATION POUR LE TRAITEMENT D'INFECTIONS DE MAMMIFÈRES PROVOQUÉES PAR MYCOBACTERIUM OU CHAMPIGNONS

(30) Priority: 17.10.2022 IT 202200021339
(43) Date of publication of application: 27.08.2025
(73) Proprietor: Università Degli Studi di Pavia, 27100 Pavia (IT); Fondazione per la Ricerca sulla Fibrosi Cistica - ETS, 37126 Verona (IT)
(72) Inventor: PASCA, Maria Rosalia, 27100 PAVIA (IT); DEGIACOMI, Giulia, 27100 Pavia (IT); RIABOVA, Olga, MOSCOW, 115035 (RU); MAKAROV, Vadim, MOSCOW, 115035 (RU)
(74) Representative: Bird & Bird Società tra Avvocati S.r.l.
(86) International application number: PCT/EP2023/078712
(87) International publication number: WO 2024/083764

(56) References cited:
- SALINA ELENA G. ET AL: "Copper-related toxicity in replicating and dormant Mycobacterium tuberculosis caused by 1-hydroxy-5- R -pyridine-2(1 H )-thiones", METALLOMICS, vol. 10, no. 7, 1 January 2018 (2018-01-01), GB, pages 992 - 1002, XP093033618, ISSN: 1756-5901, DOI: 10.1039/C8MT00067K
- "Pyridine Derivatives and Related Compounds (Benzopyridines = Quinolines) ED - Wilfred Paulus", DIRECTORY OF MICROBICIDES FOR THE PROTECTION OF MATERIALS, SPRINGER, NL, 1 January 2005 (2005-01-01), pages 638 - 649, XP009101518, ISBN: 978-1-4020-2817-5
- CAMPOS DEBORA L ET AL: "Bactericidal effect of pyridine-2-thiol 1-oxide sodium salt and its complex with iron against resistant clinical isolates of", THE JOURNAL OF ANTIBIOTICS, NATURE PUBLISHING GROUP UK, LONDON, vol. 73, no. 2, 16 October 2019 (2019-10-16), pages 120 - 124, XP036968744, ISSN: 0021-8820, [retrieved on 20191016], DOI: 10.1038/S41429-019-0243-3

## Description

The present invention relates to pyridine-2-thiol 1-oxide derivatives and their use as antimicrobial agents in infectious diseases of mammals (humans and animals) caused by fungi or bacteria, in particular belonging to the Mycobacteriaceae family, especially diseases caused by *Mycobacterium abscessus* or *Mycobacterium tuberculosis.*

The *Mycobacterium abscessus* complex which encompasses three subspecies (*M. abscessus* subsp. *abscessus; M. abscessus* subsp. *bolletii* and *M. abscessus* subsp. *massiliense*) is a group of rapidly growing nontuberculous mycobacteria (NTM) [1]. In addition to being responsible for a wide range of skin and soft tissue infections [2], these bacteria are frequent opportunistic pathogens of the lung in patients with cystic fibrosis (CF) and chronic obstructive pulmonary disease (COPD) [3]. The *M*. *abscessus* complex are with the *Mycobacterium avium* complex the NTM most frequently associated with pulmonary disease. The prevalence of *M. abscessus* complex in individual with CF ranges from 5% to 20% [4-6].

*M. abscessus* infections are difficult to treat and require long-term treatment with multidrug regimen, which is complicated by intrinsic (natural) and acquired resistance mechanisms and the lack of bactericidal activity of most of the antibiotics used [7]. Other important challenges include the absence of a correlation between *in vitro* minimal inhibitory concentrations (MIC) of compounds and their *in vivo* efficacy, the ability of these bacteria to grow both intra- and extra-cellularly, and their propensity to form biofilms [8].

To date, there is no official standard drug regimen to treat *M. abscessus* pulmonary infections. Recommendations by the British Thoracic Society (BTS) [9] describe a 12+ month-long treatment consisting of an initial phase (including intravenous and oral antibiotics), followed by a continuation phase (including inhaled and/or oral antibiotics). The combination of antibiotics used in the management of *M. abscessus* infections depends on the macrolide-sensitive or macrolide-resistant phenotype of the isolates, and includes clarithromycin/azithromycin (macrolides, only in the first case), amikacin (aminoglycosides), tigecycline (tetracyclines), imipenem (β-lactam antibiotics), clofazimine (phenazines), linezolid (oxazolidinones), minocycline (tetracyclines), moxifloxacin (fluoroquinolones), and cotrimoxazole. The American Thoracic Society and the Infectious Diseases Society of America (ATS/IDSA) guidelines were updated in July 2020 [10]. It is suggested to use at least 3 active drugs among patients without macrolide resistance, and at least 4 active drugs among patients with macrolide resistance in the initial treatment phase (parenteral - amikacin, imipenem (or cefoxitin), tigecycline, and oral - azithromycin (clarithromycin), clofazimine, linezolid); and at least 2-3 active drugs for the continuation phase of therapy (azithromycin (clarithromycin), clofazimine, linezolid, inhaled amikacin). However, it is noted in this guideline that the optimal drug regimens and duration of anti-M. *abscessus* pulmonary infection therapy are not known.

It is clear that the current recommended regimens for the treatment of *M. abscessus* pulmonary infections lack efficacy. A meta-analysis by Kwak et al. [11] revealed that the treatment success rate was only 33% for *M*. *abscessus* subsp. *abscessus* and about 57% for *M. abscessus* subsp. *massiliense.* The use of azithromycin, amikacin, or imipenem was associated with better success rate in patients with *M. abscessus* subsp. *abscessus* infection, while the choice of these drugs among patients with *M. abscessus* subsp. *massiliense* did not correlate with treatment success.

Similar results were presented in a retrospective analysis of the efficacy and adverse effects of different antibiotics used in combination to treat patients with *M. abscessus* pulmonary infection by Chen et al. [12]. The treatment success rate in patients with *M. abscessus* subsp. *massiliense* was higher (81%) compared to that among patients with *M. abscessus* subsp. *abscessus* (33%), and this rate was 45% for all observed cases of *M. abscessus* complex lung infection. Treatments with drug combinations that included amikacin, imipenem, linezolid, or tigecycline were overall more successful, but these drug combinations did not exert the same beneficial effects on patients infected with *M. abscessus* subsp. *massiliense.* It was also found that clarithromycin was more commonly associated with treatment failure than treatment success (85 vs. 71%, respectively) in patients infected with *M. abscessus* subsp. *abscessus.* In addition, adverse side events were observed in the majority of patients (79% of the cases) and included gastrointestinal distress (48%) mostly caused by tigecycline, ototoxicity (23%) associated with amikacin treatment; and myelosuppression (10%) caused mainly by linezolid. The failure of currently used drugs emphasizes the urgent need for novel antibacterial agents with new mechanisms of action, as well as for new, more effective, antibiotic combinations. To date, only few molecules are in clinical development for the treatment of *M. abscessus* pulmonary infections. In a phase 2 open-label pilot study of inhaled NO (160 ppm) administered to CF patients with refractory *M. abscessus* lung infection, the treatment was well-tolerated and safe, but did not achieve statistical significance in secondary clinical outcomes, including *M. abscessus* burden in airways [13]. The application of high-dose nitric oxide (240 ppm) in compassionate-use treatment led to adverse symptoms in a 24-year-old CF patient with pulmonary *M. abscessus* [14]. In a Phase 2 open-label study of tigecycline-containing regimens for the treatment of *M. abscessus* and *Mycobacterium chelonae* pulmonary infections, only 16 out of 36 patients (44.4%) showed clinical improvement [15]. In addition, treatment with this drug is associated with adverse events in more than 90% of participants (nausea, vomiting, fever and diarrhea being the most common). The clinicaltrials.gov website mentions two ongoing studies on the use of liposomal amikacin inhalation in the treatment of *M. abscessus* lung disease (ClinicalTrials.gov, NCT03038178 and NCT04163601), but results are not yet available.

There are two main strategies to enhance the pipeline of effective anti-*M*. *abscessus* drugs. The first strategy consists in old drug repurposing, or the study of marketed drugs for new therapeutic applications. This approach is particularly interesting in the area of antibacterial where the emergence of resistance often outpaces drug development, given that the repurposing of previously approved drugs can shorten development time and lower costs. The second strategy is the common drug development process, which involves the discovery of novel chemical entities from target identification to lead optimization, full-scale preclinical studies and clinical trial. This approach is benefiting from the development of new anti-*M*. *tuberculosis* compounds which can simultaneously be tested against other mycobacterial pathogens, including the *M. abscessus* complex.

Several recent reviews have considered the challenges and opportunities associated with drug repurposing for the treatment of *M. abscessus* pulmonary infections [16]. Only a few articles, in contrast, discuss the novel compounds that are being developed against this emerging pathogen [17-19].

Salina Elena G. et al., 2018 disclose the use of free bases of N-oxide pyridine-2-thione derivatives in the treatment of *Mycobacterium tuberculosis,* albeit not the use of their salts.

The use of sodium pyrithione as antimicrobial agent against *Mycobacterium tuberculosis, Candida albicans, Penicillium notatum* and *Aspergillus niger* is reported in the handbook *"Directory of microbicides for the protection of materials"* and in the paper Campos D. et al., 2019. However, the disclosed compounds are not substituted at 5-position of the pyridine moiety and are inactive or have a little activity against *Mycobacterium abscessus.*

Therefore, there is an urgent need of antimicrobial drugs with specific high anti-M̅. *abscessus* activity to overcome problems concerning antibiotic resistance and drug intolerance.

The authors of the present invention now discovered a promising class of compounds with specific activity against *Mycobacterium spp.* that address and solve the above mentioned technical problems.

Surprisingly the compounds of the invention exhibit strong antimicrobial activity, especially against *Mycobacterium abscessus* or *Mycobacterium tuberculosis.* Thus, the compounds of the invention are useful for the treatment of microbial infections in humans and in animals.

Therefore, it is an object of the present invention a compound of formula (I): 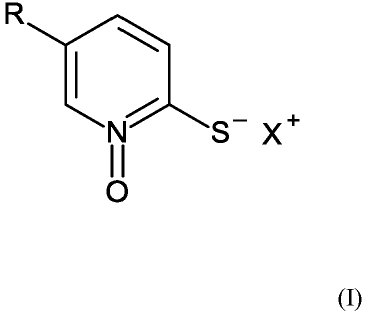
wherein R is selected from the group consisting of COOR¹, CONR₂² or CF₃, wherein
R¹ is a linear C₁-C₃ alkyl group and R² is selected between H and C₁-C₃ alkyl group;
wherein X is selected from the group consisting of Na, Li, K or Ca;
and crystalline forms thereof;
for use in the treatment or prevention of infections caused by Mycobacteria or fungi in a subject in need of such treatment.

According to a preferred embodiment of the invention, said R substituent is selected between the group consisting of 5-methoxycarbonyl, 5-ethoxycarbonyl and 5-[(dimethylamino)carbonyl].

In a preferred embodiment of the present invention, X is Na, preferably in combination with any of the above preferred meaning of R substituent.

In one of the most preferred embodiment of the invention the compound is sodium 5-(alkcarbonyl)pyridine-2-thiolate 1-oxide.

In another preferred embodiment said infection caused by Mycobacteria is caused by *Mycobacterium abscessus* or *Mycobacterium tuberculosis.*

According to a further preferred embodiment said infection caused by fungi is caused by *Sporobolomyces salmonicolor, Candida albicans, Penicillium notatum* or *Aspergillus niger.*

The invention is further directed to the use of a compound of formula (I), for the treatment or prevention of infections caused by Mycobacteria or fungi in a subject in need of such treatment alone or in combination with other active principle having antimicrobial activity.

Another object of the present invention is a pharmaceutical composition comprising a compound of formula (I) as active principle together with pharmaceutically acceptable adjuvant and excipients for use in the treatment or prevention of infections caused by Mycobacteria or fungi in a subject in need of such treatment.

According to a preferred embodiment the route od administration of the pharmaceutical compositions of the invention is systemic or topical.

The compounds of formula (I) of the present invention may be formulated in a wide variety of oral administration dosage forms and carriers. Oral administration can be in the form of tablets, coated tablets, dragée, hard and soft gelatin capsules, solutions, emulsions, syrups, or suspensions. Compounds of the present invention are efficacious when administered by other routes of administration including continuous (intravenous drip) topical parenteral, intramuscular, intravenous, subcutaneous, transdermal (which may include a penetration enhancement agent), buccal, nasal, nebulizer inhalation and suppository administration, among other routes of administration.

As a preferred embodiment said pharmaceutical compositions may be in the form of saline solutions for injection, intranasal spray or nebulizer.

The compounds of the invention may be used in dosages from 0.01 - 1000 mg/kg body weight, preferably from 1-50 mg/kg body weight.

The invention further relates to the compound of formula (I) sodium 5-(alkcarbonyl)pyridine-2-thiolate 1-oxide for use in medical field.

It is a further object of the present invention the compound of formula (I) sodium 5-(alkcarbonyl)pyridine-2-thiolate 1-oxide for use in medical field as antibacterial or antifungal agent.

In a preferred embodiment the compound is used against infection caused by Mycobacteria (preferably by *Mycobacterium abscessus* or *Mycobacterium tuberculosis*) or fungi such as *Sporobolomyces salmonicolor, Candida albicans, Penicillium notatum* or *Aspergillus niger.* Moreover, said infection may be mediated by other bacteria such as *Escherichia coli, Acinetobacter baumanii* and *Staphylococcus aureus* (see below Table 2).

The present invention will now be described for illustrative, but non-limiting purposes, according to a preferred embodiment with particular reference to the attached figures, in which:
- Figure 1 shows the concentration in plasma of compound 2 after single 100 mg/kg per os administration;
- Figure 2 shows the results of stability study carried out with 5-(ethoxycarbonyl) pyridine-2-thiolate 1-oxide (base form);
- Figure 3 shows the results of stability study carried out with sodium 5-(ethoxy carbonyl)pyridine -2-thiolate 1-oxide (salt form).

The following non-limiting examples are now provided for a better illustration of the invention, in which different silk fibroin-based scaffolds were tested and compared.

### EXAMPLE 1: Synthetic reaction scheme

### Starting materials

All reagents and solvents were purchased from commercial suppliers and used without further purification. ¹H and ¹³C spectra were measured on Bruker AC-500 (500 MHz, ¹H) or Bruker AC-200 (75 MHz, ¹³C).

Chemical shifts were measured in DMSO-d6 or CDCl₃, using tetramethylsilane as an internal standard, and reported as units (ppm) values. The following abbreviations are used to indicate the multiplicity: s, singlet; d, doublet; t, triplet; m, multiplet; dd, doublet of doublets; brs, broad singlet; brm, broad multiplet.

Mass spectra were recorded on Finnigan MAT INCO 50 mass spectrometer (EI, 70 eV) with direct injection.

The purity of the final compounds was analyzed on Agilent 1290 Infinity II HPLC system coupled to Agilent 6460 triple-quadrupole mass spectrometer equipped with an electrospray ionization source. The chromatographic separation was carried out on Agilent Eclipse Plus C18 RRHD column (2.1 × 50 mm, 1.8 µm) at 40 °C, sample injection volume was 0.2 µL. The mobile phase comprising 0.1% formic acid/water (A), and 0.1% formic acid and 85% acetonitrile/water (B) was programmed with gradient elution (0.0-3.0 min, 60% B; 3.0-4.0 min, 60% to 97% B; 4.0-6.0 min, 97% B; 6.0-6.1 min, 97% to 60% B) at a flow rate of 0.4 ml/min. The mass spectrometric detection was operated in positive ion mode. Optimal parameters were: capillary voltages of 3500 V, a nebulizer pressure of 35 psi, a gas temperature of 350°C, a gas flow rate of 12 L/min. All final compounds are > 95% pure.

Melting points were determined on Electrothermal 9001 (10°C per min).

Merck KGaA silica gel 60 F254 plates were used for analytical thin-layer chromatography.

Column chromatography was performed on Merck silica gel 60 (70-230 mesh). Yields refer to purified products.

The starting materials and the intermediates of the synthetic reaction schemes also can be isolated and purified if desired using conventional techniques, including but not limited to, filtration, distillation, crystallization, chromatography, and the like. Such materials can be characterized using conventional means, including physical constants and spectral data.

Examples of representative compounds encompassed by the present invention and within the scope of the invention are provided in the following Examples A-L.

All compounds were synthesized according below Scheme 1 wherein corresponding 2-chloropyridine was oxidized by reacting with freshly prepared solution of H₂O₂/OC(NH₂)₂ in chloroform with formation of pyridine N-oxide, which can be transformed to 2-thiopyridine by two methods:
A) reaction of 2-chloropyridine N-oxide with large excess of sodium hydrosulfide;
B) synthesis of pyridin-2-yl imidothiocarbamate as intermediate with follow its treatment by sodium hydrogen carbonate. Pyridine-2-thiol 1-oxide derivatives easy form corresponding salts with metal hydroxides and these final compounds are solid, white and water soluble compounds.

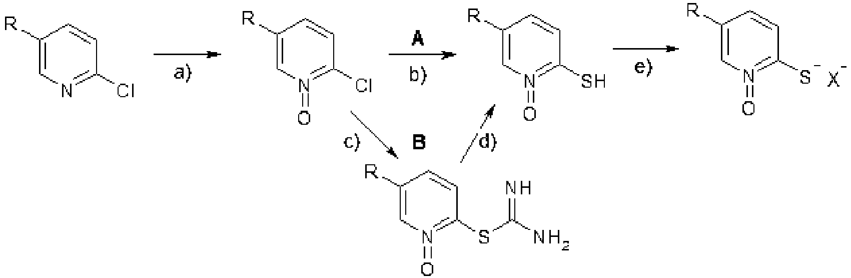

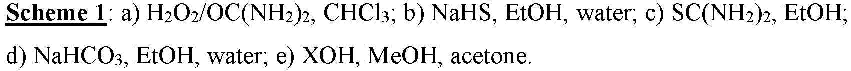

Step of the synthesis according to Scheme 1 are herein explained in more detail:
Step a) A solution of corresponding pyridine (1 mmol) in chloroform was treated by dry UHP (H₂O₂/OC(NH₂)₂ (2 mmol) at 0°C. Trifluoroacetic acid anhydride was then slowly added to the reaction mixture (the reaction is exothermic). The reaction was followed by TLC until completion at room temperature. The reaction mixture was dissolved by water, formed precipitate was filtered off and washed by water. The desired pyridine N-oxide was recrystallized preferably from ethanol.

Step b) A solution of pyridine N-oxide derivative (1.0 mmol) in ethanol was slowly treated by drop by solution of sodium hydrosulfide (5-7 mmol) at room temperature and was stored 2-3 hours at this temperature. The reaction mixture was cooled, diluted by water and formed solid was filtered off. The thiopyridine derivative was recrystallized preferably from ethyl acetate or ethanol.

Step c) A solution of pyridine N-oxide derivative (1.0 mmol) in acetone was treated by thiourea (1/3 mmol) and refluxed for 2-3 hours. The reaction mixture was evaporated in vacuum till ¼ of the volume and cooled at -18°C for 6 hours. The formed precipitate was filtered off and residue was dissolved in small volume of water. This solution was treated by water solution of sodium hydrogen carbonate and formed sediment of pyridin-2-yl imidothiocarbamate derivative was filtered off. The compound was recrystallized preferably from ethyl acetate or ethanol.

Step d) A solution of pyridin-2-yl imidothiocarbamate derivative (1 mmol) in 20 ml of ethanol was treated by water solution of sodium hydrogencarbonate (2 mmol) at room temperature. The reaction mixture was stored for 3 hours at 60°C, cooled and treated by 0.1N hydrochloric acid water solution until pH~2. Formed residue was collected, dried and pyridine-2-thiol 1-oxide derivative was recrystallized from ethanol.

Step e) A solution of pyridine-2-thiol 1-oxide derivative (1 mmol) in ethanol was treated by drop by alkali hydroxide (1 mmol) solved in minimal volume of water. Sediment was collected and recrystallized from methanol or ethanol to give aim corresponding alkaline salt of 5-(R)-pyridine-2-thiolate 1-oxide as an off-white solid.

All compounds were obtained at a purity of not less than 98% in sufficient amounts. The confirmation of the structure and investigation of the physicochemical properties of all synthesized compounds were performed by various modern methods described in the following section referring to "Examples A-L".

### Examples A-L

### A. Ethyl 6-mercaptonicotinate 1-oxide (compound 1)

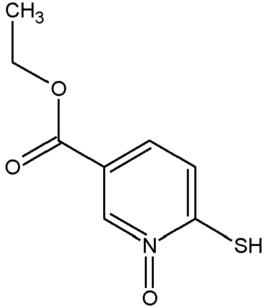

Yield 76%. Mass (EI), m/z (Rel. int. (%)): 199.2298 [M] (73). Anal. Calcd. for C₈H₉NO₃S: (%) C, 48.23; H, 4.55; N, 7.03. Found (%) C, 48.07; H, 4.36; N, 7.18. 1H NMR (DMSO-d₆): 8.74 (s, 1H, CH), 7.68 (d, 1H, *J* = 9.8 Hz, CH), 7.59 (d, 1H, *J* = 9.8 Hz, CH), 4.28 (q, 2H, *J* = 7.2 Hz, CH₂), 1.28 (t, 3H, *J* = 6.9 Hz, CH₃) ppm. ¹³C NMR (50 MHz, DMSO) δ 164.47, 162.64, 137.28, 134.16, 125.91, 121.08, 61.07, 14.30 ppm.

### B. Sodium 5-(ethoxycarbonyl)pyridine-2-thiolate 1-oxide (compound 2)

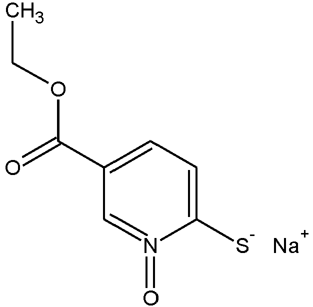

Yield 64%. Mass (EI), m/z (Rel. int. (%)): 198.2201 [M] (79). Anal. Calcd. for C₈H₈NNaO₃S: (%) C, 43.44; H, 3.65; N, 6.33. Found (%) C, 43.35; H, 3.67; N, 6.28. ¹H NMR (DMSO-d₆): 8.82 (s, 1H, CH), 7.62 (d, 1H, *J* = 9.8 Hz, CH), 7.51 (d, 1H, *J* = 9.8 Hz, CH), 4.23 (q, 2H, *J* = 7.2 Hz, CH₂), 1.17 (t, 3H, *J =* 6.9 Hz, CH₃)ppm. ¹³C NMR (50 MHz, DMSO) δ 169.71, 164.58, 137.76, 136.33, 132.17, 125.71, 61.07, 14.31 ppm.

### C. Methyl 6-mercaptonicotinate 1-oxide (compound 3)

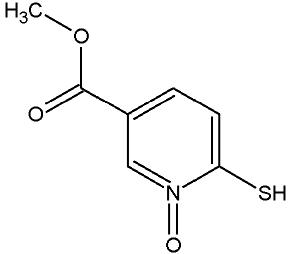

Yield 73%. Mass (EI), m/z (Rel. Int. (%)): 185.2014 [M] (57). Anal. Calcd. for C₇H₇NO₃S: (%) C, 45.40; H, 3.81; N, 7.56. Found (%) C, 45.17; H, 4.01; N, 7.59. ¹H NMR (DMSO-d6): 8.74 (s, 1H, CH), 7.69 (d, 1H, J = 9.8 Hz, CH), 7.57 (d, 1H, J = 9.8 Hz, CH), 3.77 (s, 3H, OCH3) ppm. 13C NMR (50 MHz, DMSO) δ 164.61, 162.58, 137.79, 133.83, 125.87, 121.92, 52.23 ppm.

### D. Sodium 5-(methoxycarbonyl)pyridine-2-thiolate 1-oxide (compound 4)

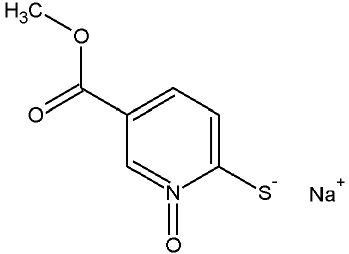

Yield 86%. Mass (EI), m/z (Rel. Int. (%)): 184.1935 [M] (65). Anal. Calcd. for C₇H₆NNaO₃S: (%) C, 40.58; H, 2.92; N, 6.76. Found (%) C, 40.49; H, 3.03; N, 6.79. ¹H NMR (DMSO-d₆): 8.70 (s, 1H, CH), 7.68 (d, 1H, *J* = 9.8 Hz, CH), 7.51 (d, 1H, *J* = 9.8 Hz, CH), 3.75 (s, 3H, OCH₃) ppm. ¹³C NMR (50 MHz, DMSO) δ 172.82, 164.81, 138.10, 136.67, 132.17, 125.61, 52.29 ppm.

### E. Sodium 5-(trifluoromethyl)pyridine-2-thiolate 1-oxide (compound 5)

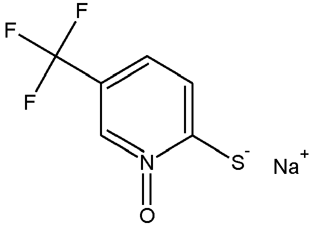

Yield 80%. Mass (EI), m/z (Rel. Int. (%)): 194.1554 [M] (79). Anal. Calcd. for C₆H₃F₃NNaOS: (%) C, 37.12; H, 1.56; N, 7.21. Found (%) C, 37.20; H, 1.49; N, 7.27. ¹H NMR (DMSO-d₆): 8.41 (s, 1H, CH), 7.97 (d, 1H, *J =* 9.7 Hz, CH), 7.67 (d, 1H, *J =* 9.7 Hz, CH) ppm. ¹³C NMR (50 MHz, DMSO) δ 171.51, 134.12, 132.57, 132.34, 131.26, 131.08, 127.41, 123.34(q), 52.29 ppm.

### F. Potassium 5-(aminocarbonyl)pyridine-2-thiolate 1-oxide (compound 6)

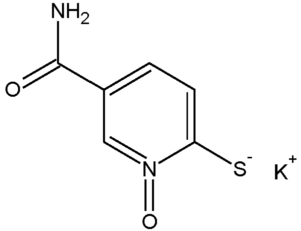

Yield 93%. Mass (EI), m/z (Rel. Int. (%)): 169.1822 [M] (64). Anal. Calcd. for C₆H₅KN₂O₂S: (%) C, 34.60; H, 2.42; N, 13.45. Found (%) C, 34.82; H, 2.56; N, 13.51. ¹H NMR (DMSO-d₆): 8.85 (s, 1H, CH), 8.04 (d, 1H, *J* = 9.6 Hz, CH), 7.73 (d, 1H, *J* = 9.6 Hz, CH), 5.67 (br s, 2H, NH₂) ppm. ¹³C NMR (50 MHz, DMSO) δ 172.53, 170.60, 140.98, 134.61, 133.53, 129.44 ppm.

### G. Potassium 5-(piperidin-1-ylcarbonyl)pyridine-2-thiolate 1-oxide (compound 7)

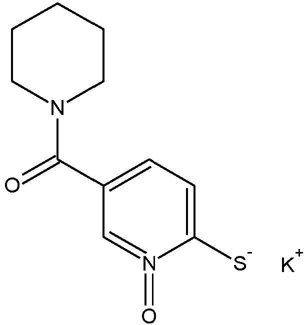

Yield 87%. Mass (EI), m/z (Rel. int. (%)): 237.2992 [M] (60). Anal. Calcd. for C₁₁H₁₃KN₂O₂S: (%) C, 47.80; H, 4.74; N, 10.14. Found (%) C, 47.61; H, 4.61; N, 10.23. ¹H NMR (DMSO-d₆): 8.67(s, 1H, CH), 7.99 (d, 1H, *J =* 9.7 Hz, CH), 7.69 (d, 1H, *J* = 9.7 Hz, CH), 3.35 (m, 4H, N(CH₂)₂), 1.93 (m, 4H, 2CH₂), 1.37 (m, 2H, CH₂) ppm. ¹³C NMR (50 MHz, DMSO) δ 170.03, 167.55, 141.99, 133.92, 132.79, 132.18, 46.70, 26.15, 24.85 ppm.

### H. 6-Mercapto-N,N-dimethylnicotinamide 1-oxide (compound 8)

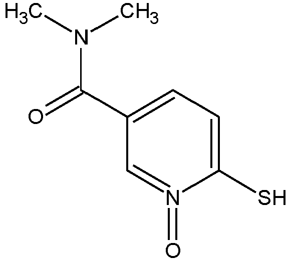

Yield 65%. Mass (EI), m/z (Rel. int. (%)): 198.2433 [M] (34). Anal. Calcd. for C₈H₁₀N₂O₂S: (%) C, 48.47; H, 5.08; N, 14.13. Found (%) C, 48.53; H, 5.14; N, 14.22. ¹H NMR (DMSO-d₆): 8.67 (s, 1H, CH), 8.03 (d, 1H, *J* = 9.7 Hz, CH), 7.67 (d, 1H, *J* = 9.7 Hz, CH), 3.12 (s, 6H, N(CH₃)₂) ppm. ¹³C NMR (50 MHz, DMSO) δ 167.87, 163.13, 135.71, 132.59, 131.06, 122.74, 35.62, 34.20 ppm.

### I. Sodium 5-[(dimethylamino)carbonyl]pyridine-2-thiolate 1-oxide (compound 9)

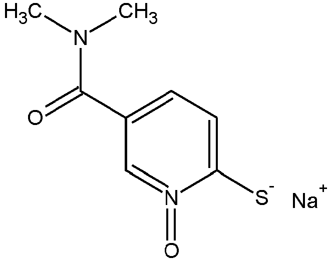

Yield 79%. Mass (EI), m/z (Rel. int. (%)): 197.2353 [M] (47). Anal. Calcd. for C₈H₉N₂NaO₂S: (%) C, 43.63; H, 4.12; N, 12.72. Found (%) C, 43.57; H, 4.11; N, 12.68. ¹H NMR (DMSO-d₆): 8.73 (s, 1H, CH), 8.00 (d, 1H, *J* = 9.7 Hz, CH), 7.71 (d, 1H, *J* = 9.7 Hz, CH), 3.12 (s, 6H, N(CH₃)₂) ppm. ¹³C NMR (50 MHz, DMSO) δ 172.41, 167.57, 142.69, 133.92, 133.45, 132.39, 37.60, 34.27 ppm.

### L. Sodium 5-(isopropoxycarbonyl)pyridine-2-thiolate 1-oxide (compound 10)

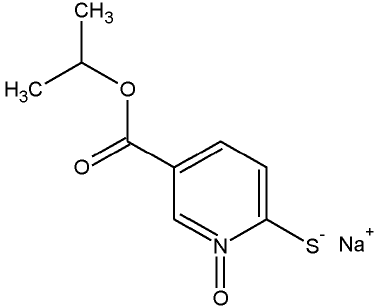

Yield 63%. Mass (EI), m/z (Rel. int. (%)): 212.2466 [M] (53). Anal. Calcd. for C₉H₁₀NNaO₃S: (%) C, 45.95; H, 4.28; N, 5.95. Found (%) C, 45.87; H, 4.33; N, 6.04. ¹H NMR (DMSO-d₆): 8.69 (s, 1H, CH), 8.16 (d, 1H, *J* = 9.7 Hz, CH), 7.49 (d, 1H, *J* = 9.7 Hz, CH), 4.93 (m, 1H, CH), 1.14 (d, 6H, J = 4.2 Hz, 2CH₃) ppm. ¹³C NMR (50 MHz, DMSO) δ 172.82, 164.50, 137.42, 135.99, 132.17, 125.75, 69.65, 17.98 ppm.

### EXAMPLE 2: In vitro activity of the compounds of formula (I)

To determine the *in vitro* activity of compounds (MIC₉₀) against *Mycobacterium abscessus* ATCC 19977, the resazurin reduction microplate assays (REMA) is performed. Briefly, serial 2-fold dilutions of drug are prepared in 96-well microtiter plates (Fluoronunc, Thermo Fisher, Waltham, MA, USA) in 100 µL of Middlebrook 7H9 medium (Difco), supplemented with 10% OADC without addition of Tween 80. Then, *M. abscessus* log-phase cultures were diluted (OD₆₀₀= 0.02) and added in the 96-well microtiter plate. Growth controls containing no compound and sterile controls without inoculum are also included. Also, a compound known to be active against *M*. *abscessus* growth is included as positive control. After 1 day of incubation at 37°C, 10 µL of resazurin (0.025% w/v) is added to each well, and bacterial viability is assessed after a further 24 h of incubation using a Fluoroskan^{™} Microplate Fluorometer (Thermo Fisher Scientific, Waltham, MA, USA; excitation= 544 nm, emission= 590 nm). Bacterial viability was calculated as a percentage of resazurin turnover in the absence of compound (MIC₉₀). is used as positive control. Results are expressed as the average of at least three independent replicates and presented on the following Table 1.

**Table 1. In vitro activity of compounds (MIC₉₀) against Mycobacterium abscessus ATCC 19977.**

| Example # | MIC in *M. abscessus* ATCC 19977 (mg/ml) |
|---|---|
| Compound 1 | 0.125 |
| Compound 2 | 0.125 |
| Compound 3 | 0.25 |
| Compound 4 | 0.25 |
| Compound 5 | 0.25 |
| Compound 6 | 0.5 |
| Compound 7 | 1.0 |
| Compound 8 | 0.5 |
| Compound 9 | 0.5 |
| Compound 10 | 0.5 |
| Control | |

By using the same procedure, the compound according to Example B (compound 2) was tested on broad spectrum of microorganisms and the corresponding results are presented in the below Table 2.

**Table 2. In vitro activity of compound 2 (MIC₉₀) on broad spectrum of microorganisms.**

| **Strains** | **Genotype** | **MIC to Compound 2 (mg/ml)** |
|---|---|---|
| *M. abscessus* ATCC 19977 | Wild-type | 0.25 |
| *M. bolletii* 1999-0888 | Wild-type | 0.5 |
| *M. massiliense* 2005-0524 | Wild-type | 0.5 |
| *M. abscessus* MDR clinical isolate 1 | Resistant to amikacin, clarithromycin, doxycycline, bedaquiline, ciprofloxacin, erytromicin, meropenem, econazole, ethambutol, ethionamide, lansoprazole, pristinamicin, rifampicin, rifapentine, SQ109, sutezolid, thiacetazone. | 0.5 |
| *M. abscessus* MDR clinical isolate 2 (from CF patient) | Resistant to amikacin, amoxicillin, clavulanic acid, cefepime, cefoxitine, ceftriaxone, ciprofloxacin, doxyciclin, imipenem, linezolid, minocycline, moxifloxacin, tobramycin, trimethoprim/sulfam | 0.5 |
| *M. abscessus* clinical isolate n. 6 | Resistant to clarithromycin, moxifloxacin, doxycycline, linezolid | 0.5 |
| *M. abscessus* clinical isolate n. 7 | Resistant to clarithromycin, amikacin, moxifloxacin, doxycycline (Intermediate sensitivity to linezolid) | 0.5 |
| *M. abscessus* clinical isolate n. 8 | Resistant to moxifloxacin, doxycycline | 0.5 |
| *M. abscessus* clinical isolate n. 9 | Resistant to clarithromycin, moxifloxacin, doxycycline | 0.5 |
| *M. abscessus* clinical isolate n.10 | Resistant to moxifloxacin, doxycycline | 0.5 |
| *M. avium* subsp. *avium* Chester ATCC15769 | Wild-type | 0.5 |
| *M. avium* clinical isolate n.1 | Resistant to linezolid (Intermediate sensitivity to Moxifloxacin) | 0.5 |
| *M. avium* clinical isolate n.2 | Resistant to linezolid (Intermediate sensitivity to Moxifloxacin) | 0.5 |
| *M. avium* clinical isolate n.3 | Resistant to linezolid (Intermediate sensitivity to Moxifloxacin) | 0.5 |
| *M. avium* clinical isolate n.4 | Resistant to linezolid and Moxifloxacin | 0.5 |
| *M. tuberculosis* H37Rv | Wild-type | 0.025 |
| IC1 *M. tuberculosis* MDR clinical isolate | Resistant to STR, INH, RIF, EMB | 0 0.025 |
| IC2 *M. tuberculosis* MDR clinical isolate | Resistant to STR, INH, RIF, EMB, PYR, ETH, CM | 0.025 |
| *M. bovis* BCG Pasteur | Wild-type | 0.0625 |
| *M. smegmatis mc2155* | Wild-type | 1 |
| *Acinetobacter baumannii* | | 4 |
| *Escherichia coli* ATCC 25922 | | 16 |
| *Klebsiella pneumoniae* | | 128 |
| *Pseudomonas aeruginosa* PAO1 | | ≥ 256 |
| *Pseudomonas aeruginosa* PAO1 | | 1 (in presence of efflux inhibitor) |
| *Staphylococcus aureus* ATCC 25923 | | 8 |
| MRSA *Staphylococcus aureus* clinical isolate | | 8 |
| *Burkholderia cenocepacia K56-2* | | 128 |

Antifungal agar diffusion assays were performed as previously published [20].

The well diffusion test was performed using Casitone agar. The inoculum used was prepared using the *Candida* or *Aspergillus* strains from a 24-hour culture on Sabouraud dextrose agar, a suspension was made in a sterile saline solution (0.85%). The turbidity of the suspension was adjusted with a spectrophotometer at 530 nm to obtain a final concentration to match that of a 0.5 McFarland standard (0.5-2.5 × 10³). 20 mL of Bacto-casitone were melted, cooled to 55°C and then inoculated with 1 mL of the organism suspension. The inoculated agar was poured into the assay plate (9 cm in diameter) and allowed to cool down on a leveled surface. Once the medium had solidified, well 4 mm in diameter, were cut out of the agar, and 20 µL water solution of the tested compound was placed into well and incubated at 35°C for 24 hours. The end point of the diameters of the clear zone of inhibition of growth for the azoles, it was measured after 48 hours. A dramatic effect of tested compounds was observed (see below Table 3).

**Table 3**

| **Strains** | **Inhibition Zone of Test Microorganism (mm)** | | | | |
|---|---|---|---|---|---|
| | Compound 1 | Compound 2 | Compound 5 | Compound 7 | Amph.B |
| *Sporobolomyces salmonicolor* | 43 | 46 | 40 | 43 | 15 |
| *Candida albicans H8* | 56 | 53 | 49 | 52 | 22 |
| *Penicillium notatum* | 17 | 15 | 15 | 15 | 12 |
| *Aspergillus niger* | 26 | 28 | 22 | 23 | 17 |

### EXAMPLE 3: Acute and subacute toxicity of compound 2

Acute and subacute toxicity of compound 2 was experimentally studied for intragastric administration in white mice. Test drug is white crystals soluble in water. The compound was solubilized in saline to be administrated to animals.

### Acute toxicity

Experimental animals: 35 C57BL/6 male white mice, 7 weeks old, weight 20-22 g. The following experimental procedure was used:
- administration route of the compound: per os;
- number of doses: 5;
- dosage: 100-200-250-300-500-600-750 mg/kg;
- volume introduced into 0.3 ml/20 g (15 ml/kg);
- data recorded: behavior, weight changes, rectal temperature, mortality.

### Results

During 14 days observation period compound 2 in doses lower than 300 mg/kg did not cause changes in the general state and behavior of the mice, it did not affect motoric and reflex activity, active and calm cycles, grooming, food consumption, there were no cases of animal death.
LD₁₆ = 396.07
LD₅₀ = 455.78±21,79
LD₈₄ = 515.48
LD₁₀₀ = 545.33

### Subacute toxicity

Evaluation of subacute toxicity of compound 2 observation time after 14 days per oral administration.

Experimental animals: 14 C57BL/6 male white mice, 7 weeks old, weight 20-22 g. The following experimental procedure was used:
- administration route of the compound : per os;
- dosage 50 mg/kg once a day for 14 days;
- volume introduced into 0.3 ml/20 g (15 ml/kg);
- data recorded: behavior, weight changes, rectal temperature, mortality.

**Results.** 14 days of treatment with the compound 2 in a dosage of 50 mg/kg did not cause changes in the general state and behavior of the mice, did not affect motoric and reflex activity, active and calm cycles, grooming, food consumption, there were no cases of animal death. No significant differences in the mass of mice of the experimental and control groups were found (see below Table 4).

**Table 4**

| No mice | Weight of individual mice, gramm | | | |
|---|---|---|---|---|
| | Control group | | Experimental group | |
| | 13.01.2021 | 28.01.2021 | 13.01.2021 | 28.01.2021 |
| 1 | 20.8 | 21.3 | 20.9 | 21.5 |
| 2 | 20.8 | 21.2 | 20.5 | 21.5 |
| 3 | 19.5 | 20 | 22.8 | 22.4 |
| 4 | 20.4 | 20.5 | 20.4 | 20.5 |
| 5 | 22.5 | 23.1 | 20.1 | 21.1 |
| 6 | 20.2 | 20.9 | 22.1 | 19.5 |
| 7 | | | 19.2 | 20.9 |
| 8 | | | 19.7 | 21.7 |
| M ±m | **20.7±0.4** | **21.16±0.43** | **20.71±0.4** | **21.13±0.308** |
| | | | | |

### EXAMPLE 4: Pharmacokinetic study of compound 2

The pharmacokinetics of the compound 2 has been studied white Balb/C male mice. 27 animals with average body weight of 20 g were selected for the study. The animals were kept on standard vivarium ration with dry pelleted feed before the experiment. All the test animals had free access to water but were deprived of food for 1 hour before administration of the compound. This regimen was continued for another hour after the administration.

Compound in a dose of 100 mg/kg of body weight was administered as 0.5 mL of saline solution by intragastric intubation.

The animals were euthanized by decapitation for blood and brain sampling. Blood and brain samples (3 animals per time point) were taken at 15, 30, 60, 120, 240, 360 1440 min after administration for pharmacokinetic evaluation. Blood was collected in heparinized tubes and centrifuged at 3500 RPM. Plasma was separated from formed elements and immediately frozen at -20°C in freezer. This storage continued before transferring of plasma for analysis. Serum from 6 untreated animals was used as control and for equipment calibration with compound. Mice brain was immediately frozen at -120 °C.

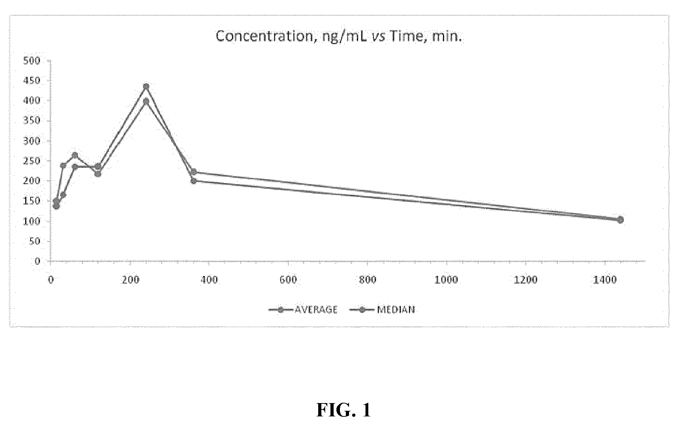

Compound 2 has high bioavailability and its plasma concentration is shown in Figure 1.

### EXAMPLE 5: Comparative stability test of the substance 5-(ethoxycarbonyl)pyridine-2-thiolate 1-oxide and sodium 5-(ethoxycarbonyl)pyridine-2-thiolate 1-oxide

The aim of the study is to investigate the physical and chemical properties of a substances at elevated temperature and to establish an experimental expiration date for the substances (saline form vs base form).

### Studied samples:

- 5-(ethoxycarbonyl )pyridine-2-thiolate 1-oxide (base form)
- sodium 5-(ethoxycarbonyl )pyridine-2-thiolate 1-oxide (compound 2 - salt form)

### Conditions for the study:

Storage temperature: (55 ± 2)°C.

### Type of packaging:

Double layer polyethylene bag. The package is placed in a bag made of laminated aluminum foil. Each package is wrapped in wrapping paper.

Intervals: substance samples were analyzed after 6, 12, 18, 23 days, which is equivalent to 6, 12, 18, 24 months when stored at 5±3 °C, respectively.

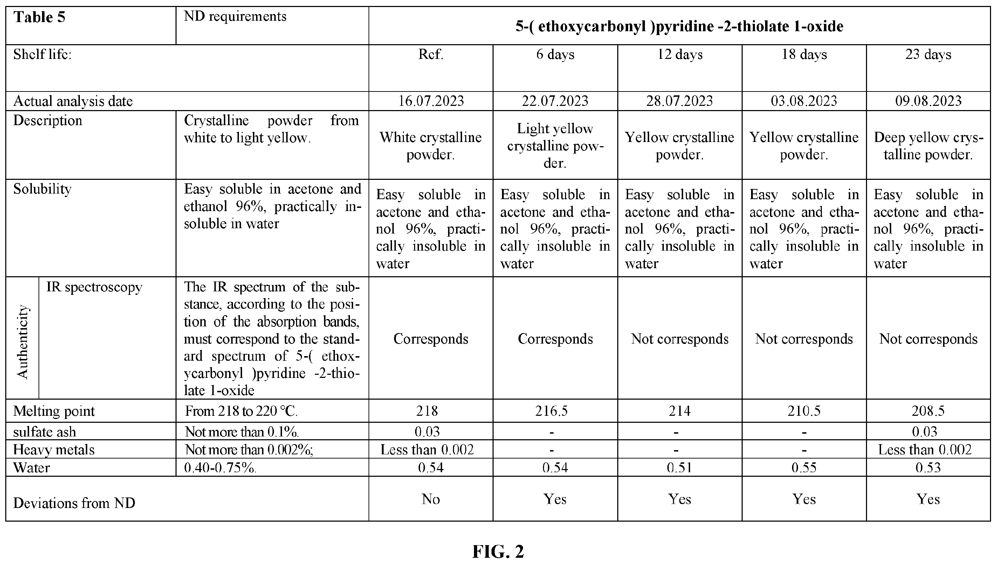

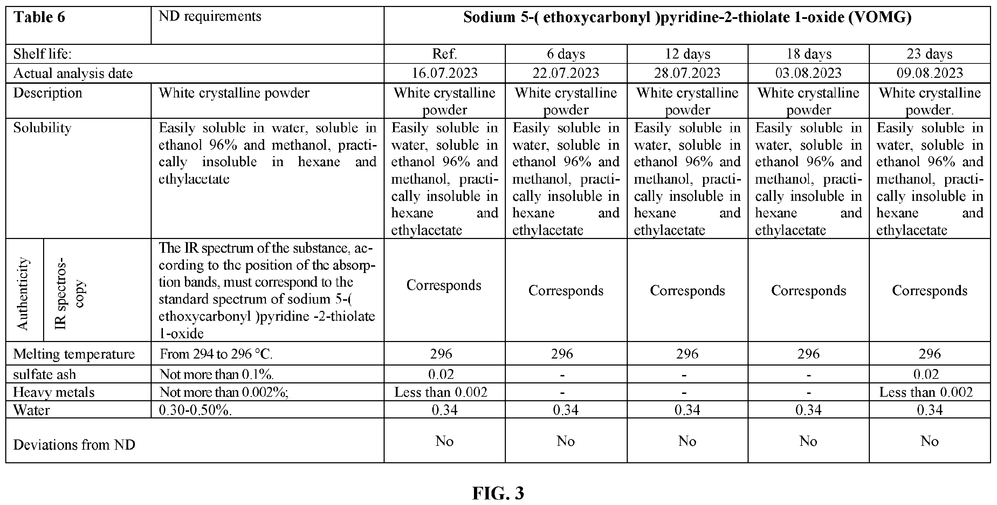

The results of the analyses are presented in Tables 5 and 6 reported in Figures 2 and 3.

According to the results of the stability study, using the accelerated aging method, we can conclude that:
- 5-(ethoxycarbonyl)pyridine-2-thiolate 1-oxide is not stable under short-term changes in storage conditions;
- sodium 5-(ethoxycarbonyl)pyridine-2-thiolate 1-oxide according of the invention is stable under short-term changes in storage conditions and experimental shelf life at a temperature of 20±3 °C is about 2 years.

Salts according to the present invention are stable solid compounds; this achievement makes them suitable for certain type of drug formulations such a nebulizer or aerosol formulation which is highly important for the treatment of lung diseases caused by Mycobacteria.

### BIBLIOGRAPHY

[1] Tortoli E, Kohl TA, Brown-Elliott BA, et al. Int J Syst Evol Microbiol. 2016, 66(11), 4471-4479. doi: 10.1099/ijsem.0.001376.
[2] Lee MR, Sheng WH, Hung CC, Yu CJ, Lee LN, Hsueh PR. Emerg Infect Dis. 2015, 21(9), 1638-1646. doi: 10.3201/2109.141634.
[3] Lopeman RC, Harrison J, Desai M, Cox JAG. Microorganisms. 2019, 7(3), 90. doi: 10.3390/microorganisms7030090.
[4] Roux AL, Catherinot E, Ripoll F, et al. J Clin Microbiol. 2009, 47(12), 4124-8. doi: 10.1128/JCM.01257-09;
[5] Esther CR Jr, Esserman DA, Gilligan P, Kerr A, Noone PG. J Cyst Fibros. 2010, 9(2), 117-123. doi: 10.1016/j.jcf.2009.12.001.
[6] Adjemian J, Olivier KN, Prevots DR. Am J Respir Crit Care Med. 2014, 190(5), 581-586. doi: 10.1164/rccm.201405-08840C.
[7] Wu ML, Aziz DB, Dartois V, Dick T. 2018, 23(8), 1502-1519. doi: 10.1016j.drudis.2018.04.001.
[8] Falkinham JO 3rd. Front Microbiol. 2018, 9, 1613. doi: 10.3389/fmicb.2018.01613.
[9] Haworth CS, Banks J, Capstick T, et al. Thorax. 2017, 72(Suppl 2), ii1-ii64. doi: 10.1136/thoraxjnl-2017-210927.
[10] Daley CL, Iaccarino JM, Lange C, et al. Clin Infect Dis. 2020, 71(4), e1-e36. doi: 10.1093/cid/ciaa241.
[11] Kwak N, Dalcolmo MP, Daley CL, et al. Eur Respir J. 2019, 54(1), 1801991. doi: 10.1183/13993003.01991-2018.
[12] Chen J, Zhao L, Mao Y, et al. Front Microbiol. 2019, 10, 1977. doi: 10.3389/fmicb.2019.01977.
[13] Bentur L, Gur M, Ashkenazi M, et al. J Cyst Fibros. 2020, 19(2), 225-231. doi: 10.1016/j.jcf.2019.05.002.
[14] [Bogdanovski K, Chau T, Robinson CJ, et al. Access Microbiology. 2020, 2(9), acmi000154. doi: 10.1099/acmi.0.000154].
[15] Wallace RJ Jr., Dukart G, Brown-Elliott BA, et al. J. Antimicrob Chemother. 2014, 69(7), 1945-1953. doi: 10.1093/jac/dku062.
[16] Egorova A., Jackson M., Gavrilyuk V., Makarov V. Medicinal Research Reviews, 2021, 41, 2350. doi: 10.1002/med.21798.
[17] Lee SFK, Laughon BE, McHugh TD, Lipman M. Curr Opin Pulm Med. 2019, 25(3), 271-280. doi: 10.1097/MCP.0000000000000570.
[18] Bento CM, Gomes MS, Silva T. 2020, 9(1), 18. doi: 10.3390/antibiotics9010018.
[19] Sethiya JP, Sowards MA, Jackson M, North EJ. Int. J Mol Sci. 2020, 21(17), 6202. doi: 10.3390/ijms21176202.
[20] Magaldi S, Mata-Essayag S, Hartung de Capriles C, Perez C, Colella MT, Olaizola C, Ontiveros Y. Int J Infect Dis. 2004 8(1):39-45. doi: 10.1016/j.ijid.2003.03.002

## Claims

1. A compound of formula (I) 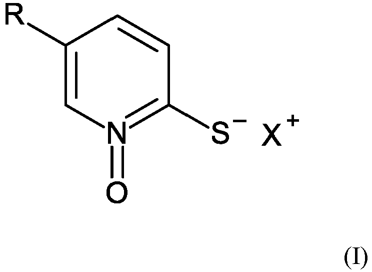
wherein R is selected from the group consisting of COOR¹, CONR₂² or CF₃,
wherein R¹ is a linear C₁-C₃ alkyl group-and R₂ is selected-between H and C₁-C₃ alkyl grou;
wherein X is selected from the group consisting of Na, Li, K, or Ca;
and crystalline forms thereof;
for use in the treatment or prevention of infections caused by Mycobacteria and/or fungi in a subject in need of such treatment.

2. A compound for use according to claim 1, wherein R is selected from the group consisting of 5-methoxycarbonyl, 5-ethoxycarbonyl and 5-[(dimethylamino)carbonyl].

3. A compound for use according to claim 1 or 2, wherein X is Na.

4. A compound for use according to anyone of claim 1-3, which is sodium 5-(alkcarbonyl)pyridine-2-thiolate 1-oxide.

5. A compound for use according to claim 4, which is sodium 5-(ethoxycarbonyl)pyridine-2-thiolate 1-oxide.

6. A compound for use according to anyone of claims 1-5, wherein said Mycobacteria is *Mycobacterium abscessus* or *Mycobacterium tuberculosis.*

7. A compound for use according to anyone of claims 1-6, wherein said fungi is selected among the group comprising *Sporobolomyces salmonicolor, Candida albicans, Penicillium notatum* and *Aspergillus niger.*

8. A compound for use according to anyone of claims 1-6 wherein said subject is a mammal, preferably a human.

9. A compound of formula (I) according to anyone of claims 1-8, for use in the treatment or prevention of infections caused by Mycobacteria and/or fungi, alone or in combination with at least one active ingredient having antimicrobial activity.

10. A pharmaceutical composition comprising a compound of formula (I) according to anyone of claims 1-5 as active principle together with pharmaceutically acceptable adjuvant and excipients for use in the treatment or prevention of infections caused by Mycobacteria or fungi in a subject in need of such treatment.

11. A pharmaceutical composition for use according to claim 10, for systemic or topical use.

12. A pharmaceutical composition for use according to anyone of the claims 10-11, in the form of saline solutions for injection, intranasal spray or nebulizer.

13. Sodium 5-(alkcarbonyl)pyridine-2-thiolate 1-oxide for use in medical field.

14. Sodium 5-(alkcarbonyl)pyridine-2-thiolate 1-oxide for use in medical field as antibacterial or antifungal agent.

15. Sodium 5-(ethoxycarbonyl)pyridine-2-thiolate 1-oxide for use in medical field.

16. Sodium 5-(ethoxycarbonyl)pyridine-2-thiolate 1-oxide for use in medical field as antibacterial or antifungal agent.

## Patentansprüche

1. Verbindung der Formel (I) 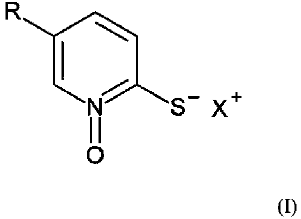
wobei R aus der Gruppe ausgewählt ist, die aus COOR¹, CONR₂² oder CF₃ besteht,
wobei R₁ eine lineare C₁-C₃ -Alkylgruppe ist und R₂ zwischen H und einer C₁-C₃-Alkylgruppe ausgewählt ist;
wobei X aus der Gruppe ausgewählt ist, die aus Na, Li, K oder Ca besteht, sowie deren kristalline Formen;
zur Verwendung bei der Behandlung oder Vorbeugung von Infektionen, die durch Mykobakterien und/oder Pilze verursacht werden, bei einem Patienten, der einer solchen Behandlung bedarf.

2. Verbindung zur Verwendung nach Anspruch 1, wobei R aus der Gruppe ausgewählt ist, die aus 5-Methoxycarbonyl, 5-Ethoxycarbonyl und 5-[(Dimethylamino)carbonyl] besteht.

3. Verbindung zur Verwendung nach Anspruch 1 oder 2, wobei X Na ist.

4. Verbindung zur Verwendung nach einem der Ansprüche 1-3, bei der es sich um Natrium-5-(alkcarbonyl)pyridin-2-thiolat-1-oxid handelt.

5. Verbindung zur Verwendung nach Anspruch 4, bei der es sich um Natrium-5-(ethoxycarbonyl)pyridin-2-thiolat-1-oxid handelt.

6. Verbindung zur Verwendung nach einem der Ansprüche 1-5, wobei es sich bei dem Mykobakterium um *Mycobacterium abscessus* oder *Mycobacterium tuberculosis* handelt.

7. Verbindung zur Verwendung nach einem der Ansprüche 1-6, wobei der Pilz aus der Gruppe ausgewählt ist, die aus *Sporobolomyces salmonicolor, Candida albicans, Penicillium notatum* und *Aspergillus niger* besteht.

8. Verbindung zur Verwendung nach einem der Ansprüche 1-6, wobei es sich bei dem Patienten um ein Säugetier, vorzugsweise einen Menschen, handelt.

9. Verbindung der Formel (I) nach einem der Ansprüche 1-8 zur Verwendung bei der Behandlung oder Vorbeugung von Infektionen, die durch Mykobakterien und/oder Pilze verursacht werden, allein oder in Kombination mit mindestens einem Wirkstoff mit antimikrobieller Aktivität.

10. Pharmazeutische Zusammensetzung, umfassend eine Verbindung der Formel (I) nach einem der Ansprüche 1-5 als Wirkstoff zusammen mit einem pharmazeutisch verträglichen Adjuvans und Hilfsstoffen zur Verwendung bei der Behandlung oder Vorbeugung von Infektionen, die durch Mykobakterien oder Pilze bei einem Patienten verursacht werden, der einer solchen Behandlung bedarf.

11. Pharmazeutische Zusammensetzung zur Verwendung nach Anspruch 10 zur systemischen oder topischen Verwendung.

12. Pharmazeutische Zusammensetzung zur Verwendung nach einem der Ansprüche 10-11 in Form von Salzlösungen zur Injektion, intranasalem Spray oder Zerstäuber.

13. Natrium-5-(alkcarbonyl)pyridin-2-thiolat-1-oxid zur Verwendung im medizinischen Bereich.

14. Natrium-5-(alkcarbonyl)pyridin-2-thiolat-1-oxid zur Verwendung im medizinischen Bereich als antibakterielles oder antimykotisches Mittel.

15. Natrium-5-(ethoxycarbonyl)pyridin-2-thiolat-1-oxid zur Verwendung im medizinischen Bereich.

16. Natrium-5-(ethoxycarbonyl)pyridin-2-thiolat-1-oxid zur Verwendung im medizinischen Bereich als antibakterielles oder antimykotisches Mittel.

## Revendications

1. Composé de formule (I) 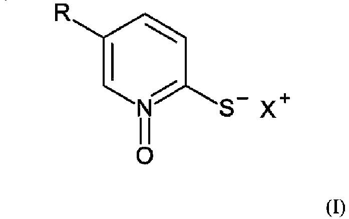
dans lequel R est choisi parmi le groupe constitué de COOR1, CONRi ou CF3, dans lequel R1 est un groupe alkyle linéaire en C1-C3 et R2 est choisi entre H et un groupe alkyle en C1-C3 ;
dans lequel X est choisi parmi le groupe constitué de Na, Li, K ou Ca ; et leurs formes cristallines ;
destiné au traitement ou à la prévention des infections causées par des mycobactéries et/ou des champignons chez un sujet nécessitant un tel traitement.

2. Composé destiné à être utilisé selon la revendication 1, dans lequel R est choisi parmi le groupe constitué du 5-méthoxycarbonyle, du 5-éthoxycarbonyle et du 5-[(diméthylamino)carbonyle].

3. Composé destiné à être utilisé selon la revendication 1 ou 2, dans lequel X représente Na.

4. Composé destiné à être utilisé selon l'une quelconque des revendications 1 à 3, qui est le 1-oxyde de 5-(alkcarbonyl)pyridine-2-thiolate de sodium.

5. Composé destiné à être utilisé selon la revendication 4, qui est le 1-oxyde de 5-(éthoxycarbonyl)pyridine-2-thiolate de sodium.

6. Composé destiné à être utilisé selon l'une quelconque des revendications 1 à 5, dans lequel ladite mycobactérie est *Mycobacterium abscessus* ou *Mycobacterium tuberculosis.*

7. Composé destiné à être utilisé selon l'une quelconque des revendications 1 à 6, dans lequel ledit champignon est choisi parmi le groupe comprenant *Sporobolomyces salmonicolor, Candida albicans, Penicillium notatum* et *Aspergillus niger.*

8. Composé destiné à être utilisé selon l'une quelconque des revendications 1 à 6, dans lequel ledit sujet est un mammifère, de préférence un être humain.

9. Composé de formule (I) selon l'une quelconque des revendications 1 à 8, destiné à être utilisé dans le traitement ou la prévention d'infections causées par des mycobactéries et/ou des champignons, seul ou en association avec au moins une substance active présentant une activité antimicrobienne.

10. Composition pharmaceutique comprenant un composé de formule (I) selon l'une quelconque des revendications 1 à 5 en tant que principe actif, associé à des adjuvants et excipients pharmaceutiquement acceptables, destinée à être utilisée dans le traitement ou la prévention d'infections causées par des mycobactéries ou des champignons chez un sujet nécessitant un tel traitement.

11. Composition pharmaceutique destinée à être utilisée conformément à la revendication 10, à usage systémique ou topique.

12. Composition pharmaceutique destinée à être utilisée conformément à l'une quelconque des revendications 10 à 11, sous forme de solutions salines pour injection, de spray intranasal ou de nébuliseur.

13. 1-oxyde de 5-(alkcarbonyl)pyridine-2-thiolate de sodium destiné à une utilisation dans le domaine médical.

14. 1-oxyde de 5-(alkcarbonyl)pyridine-2-thiolate de sodium, destiné à être utilisé dans le domaine médical comme agent antibactérien ou antifongique.

15. 1-oxyde de 5-(éthoxycarbonyl)pyridine-2-thiolate de sodium destiné à une utilisation dans le domaine médical.

16. 1-oxyde de 5-(éthoxycarbonyl)pyridine-2-thiolate de sodium, destiné à être utilisé dans le domaine médical comme agent antibactérien ou antifongique.
